# EUROPÄISCHE PATENTSCHRIFT

(11) **EP 0 977 995 B1**
(45) Veröffentlichungstag und Bekanntmachung des Hinweises auf die Patenterteilung: **02.03.2005**
(21) Anmeldenummer: 98914766.5
(22) Anmeldetag: 24.04.1998
(51) Int. Cl.: G01N 33/86

(54) **VERFAHREN ZUM FUNKTIONELLEN NACHWEIS VON STÖRUNGEN IM PROTEIN C-SYSTEM**
METHOD FOR THE FUNCTIONAL DETECTION OF DISORDERS IN THE PROTEIN C SYSTEM
PROCEDE POUR DETECTER DE MANIERE FONCTIONNELLE DES DYSFONCTIONNEMENTS DANS LE SYSTEME DE LA PROTEINE C

(30) Priorität: 25.04.1997 CH 97497; 03.09.1997 CH 206297
(43) Veröffentlichungstag der Anmeldung: 09.02.2000
(73) Patentinhaber: Pentapharm AG, 4002 Basel (CH)
(72) Erfinder: STOCKER, Kurt, CH-3981 Obergestein (CH); GEMPELER-MESSINA, Patrizia, CH-4148 Pfeffingen (CH); MÜLLER, Christian, CH-4153 Reinach (CH)
(74) Vertreter: Braun, André, jr.
(86) Internationale Anmeldenummer: PCT/CH1998/000168
(87) Internationale Veröffentlichungsnummer: WO 1998/049562

(56) Entgegenhaltungen:
- EP-A- 0 711 838
- WO-A-96/04560
- WO-A-96/15457

## Beschreibung

Die vorliegende Erfindung betrifft ein Verfahren zum sensitiven, funktionellen Nachweis von Störungen im Protein C-System (Protein C, Protein S, FV) und insbesondere zum Nachweis des aktivierten Blutgerinnungsfaktors V (FVa) mit erhöhter Stabilität gegenüber dem Abbau durch aktiviertes Protein C (APC).

Blutstillung (Hämostase) nach Gefässverletzungen resultiert aus einer Wechselwirkung zwischen Gewebe, korpuskulären Blutbestandteilen (Blutplättchen) und Proteinen der Blutflüssigkeit (Plasmagerinnungsfaktoren, Calciumionen), die zunächst zur Ausbildung eines hämostatischen Plättchenpfropfs (primäre Hämostase) und schliesslich zu dessen Konsolidierung durch Gerinnung, d.h. durch Bildung eines Netzwerks aus unlöslichem Fibrin, führt. Der physiologische Calciumgehalt des Blutes von 60 - 70 mg pro Liter ist für den optimalen Verlauf der Blutgerinnungsreaktionen von essentieller Bedeutung. Für den enzymatischen Abbau von Fibringerinnseln im Zuge der Wundheilung und der Rekanalisierung verschlossener Gefässe, sorgt das Fibrinolysesystem. Diese ineinandergreifenden Systeme werden durch Aktivatoren und Inhibitoren moduliert und befinden sich im gesunden Organismus in einem labilen Gleichgewicht Störungen dieses Gleichgewichtes können einerseits zu erhöhter Blutungsbereitschaft und andererseits zu Thromboseneigung führen. Hämostasestörungen sind Ursache oder Begleiterscheinung vieler Erkrankungen und Therapien. Dabei kann das Gleichgewicht sowohl zugunsten der Blutung als auch der Thrombose gestört sein.

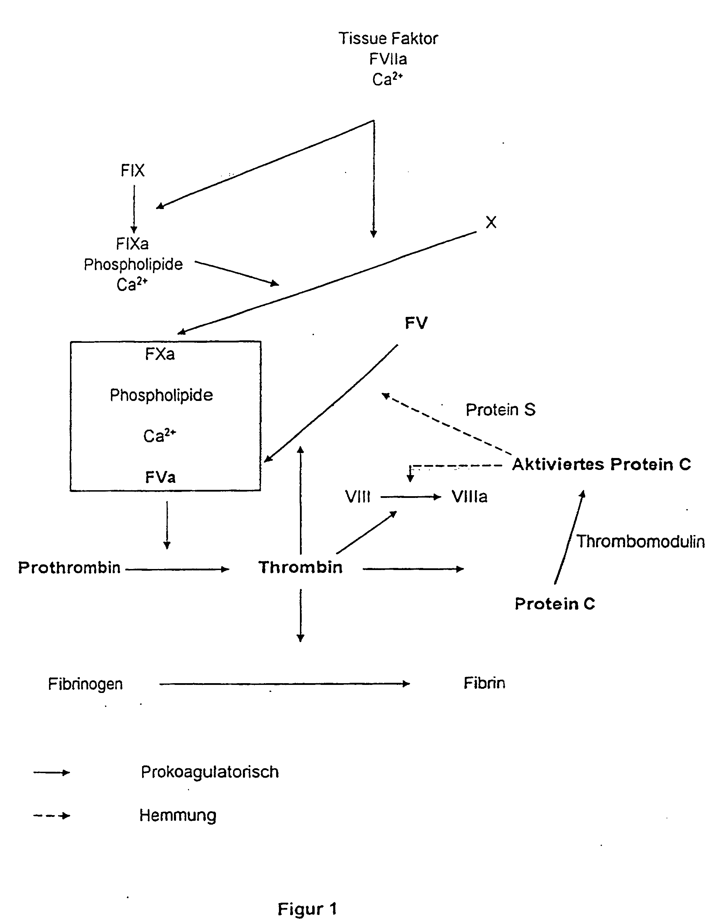

Für die vorliegende Erfindung ist der Ausschnitt der Blutgerinnungskaskade gemäss Figur 1 von Bedeutung. Eine detaillierte Übersicht über die zur Blutgerinnung führende Reaktionskaskade findet sich bei H.R. Roberts, Overview of the Coagulation Reactions, In:K.A. High and H.R. Roberts (Eds.) Molecular Basis of Thrombosis and Hemostasis,pp. 35-50, Marcel Dekker: New York, Basel, Hong Kong(1995).

Nach einer Verletzung der Gefässwand gelangt Blut mit Gewebezellen in Kontakt, welche an ihrer Oberfläche ein 50.000 Dalton grosses Glykoprotein exprimieren, welches als sogenanntes Gewebethromboplastin oder Tissue Faktor (TF) das Blutgerinnungssystem über den exogenen Weg aktiviert. An Gewebestrukturen adhärierende Blutplättchen schütten Phospholipide (Plättchenfaktor 3, Plättchenthromboplastin) aus, welche das intrinsische Blutgerinnungssystem aktivieren. TF bildet mit dem im Plasma vorhandenen Faktor (F) VII einen Komplex, welcher die Zymogene FX und FIX zu den Serinproteasen FXa und F IXa aktiviert. Über den endogenen Weg bildet F IXa zusammen mit seinem Cofaktor, FVIIIa, in Anwesenheit von Calciumionen und Phospholipiden ebenfalls einen effizienten Aktivator für FX. FXa bildet mit FVa, Calciumionen und Phospholipiden einen Komplex (Prothrombinasekomplex), welcher das Zymogen Prothrombin in katalytisch aktives Thrombin konvertiert. Das Enzym Thrombin wandelt Fibrinogen durch begrenzte Proteolyse in Fibrinmonomer um, welches spontan zu Fibrin polymerisiert.

Die Faktoren Va und VIIIa sind nicht-enzymatische Plasmaproteine, welche die Aktivierung von FX bezw. Prothrombin durch FIX resp. FXa stark beschleunigen. Die katalytische Effizienz des Kofaktors, FVa, auf die Prothrombinaktivierung wird in der Tabelle 1 erläutert. Es wird daraus ersichtlich, dass die Aktivierung von Prothrombin durch FXa, Phospholipide und Calciumionen allein durch Anwesenheit von FVa etwa 250fach beschleunigt wird.

Die Anwesenheit von Calciumionen und Phospholipiden ist für die meisten Reaktionen der Blutgerinnungskaskade eine unerlässliche Voraussetzung. Entzug von Calciumionen durch Komplexierung, Fällung oder Ionenaustausch hebt die Gerinnungsfähigkeit von Blut vollkommen auf. Diese Eigenschaft wird üblicherweise zur Gewinnung von Blutplasma für analytische oder therapeutische Zwecke dadurch genutzt, dass man frisch entnommenes Blut zur Komplexierung von Calciumionen mit Natriumcitrat vermischt, zentrifugiert und die überstehende, ungerinnbare Blutflüssigkeit von den sedimentierten Korpuskeln abdekantiert. Durch Zugabe einer physiologischen Menge eines Calciumsalzes, durch sogenannte Recalzifizierung, wird die Gerinnungsfähigkeit von Citratplasma wieder hergestellt.

**Tabelle 1.**

| Katalytische Effizienz des Prothrombinasekomplexes Prothrombinaktivierung durch den Prothrombinasekomplex | |
|---|---|
| Xa | 1 |
| Xa, Ca²⁺ | 1.7 |
| Xa, Ca²⁺, PL, | 8.3 x 10³ |
| Xa, Ca²⁺, PL, Va | 2.0 x 10⁶ |

Das antikoagulatorische Protein C System verhindert eine unkontrollierte Abwanderung aktivierter Blutgerinnungsfaktoren vom Ort der Gefässverletzung und der Blutstillung. Ähnlich wie im plasmatischen Gerinnungssystem ist auch in der Aktivierung des Protein C Systems ein zellgebundener Rezeptor, das Thrombomodulin (TM) und ein nicht enzymatischer Cofaktor, das Protein S, sowie die akzessorischen Komponenten Phospholipid und Calciumionen beteiligt. Vom Ort der Hämostase abwanderndes Thrombin wird an TM gebunden, verliert dadurch seine fibrinogenkoagulierenden Eigenschaften und wird zum spezifischen Aktivator für das Zymogen Protein C. Aktiviertes Protein C (APC) ist eine Serinproteinase, welche, potenziert durch Protein S, die Gerinnungsfaktoren FVa und FVIIIa spaltet und inaktiviert. Durch die Inaktivierung dieser Cofaktoren wird der Gerinnungsprozess stark verlangsamt, allein durch die Inaktivierung von FVa erfolgt gemäss Tabelle 1 eine 250fache Verzögerung. Für eine aktuelle Beschreibung des Protein C-Systems siehe K. Suzuki, Protein C, In: K.A. High and H.R. Roberts (Eds.) Molecular Basis of Thrombosis and Hemostasis, pp. 393-424, Marcel Dekker: New York, Basel, Hong Kong (1995).

Die biologische Bedeutung des Protein C Systems wurde 1993 durch B. Dahlbäck veranschaulicht, der beobachtete, dass sich bei Patienten mit Thromboseneigung, anders als bei gesunden Probanden, die Aktivierte Partialthromboplastinzeit (APTT, eine diagnostische Funktionskontrolle für das endogene Gerinnungssystem) nach Zusatz von aktiviertem Protein C nicht verlängert. Er definierte seine Beobachtung als "Resistenz gegen aktiviertes Protein C" (APC Resistenz). Ursache dieser Resistenz ist in 97% aller Fälle eine Punktmutation im Faktor V-Gen. Diesen genetisch vererbten Defekt findet man in etwa 5% der normalen Population und in mindestens 20% bei jüngeren Patienten mit zunächst unerklärbaren oder häufig wiederauftretenden Thromboembolien. Beim Vorliegen der Mutation kann der aktivierte Gerinnungsfaktor V nicht mehr gespalten und daher nicht inaktiviert werden. Als Folge des Versagens dieser äusserst wichtigen antikoagulatorischen Komponente des Blutgerinnungssystems können koronare Herzkrankheiten, venöse Thrombosen oder Thromboembolien auftreten. Heterozygote Defektträger haben deshalb auch ein 5-10 mal höheres Thromboserisiko gegenüber Normalpersonen und homozygote Defektträger sogar ein 50- bis 100 mal höheres Risiko.

Andere erbliche oder erworbene Mängel oder Defekte im Protein C System (Qualitative oder quantitative Mängel an Protein C oder Protein S) sind ebenfalls mit hoher Thromboseneigung assoziert.

Der Nachweis der angeborenen oder erworbenen APC-Resistenz kann durch einen funktionellen Test oder durch den direkten Nachweis der Mutation auf DNA-Ebene (Genotyp) erfolgen.

Der funktionelle Nachweis kann nach Dahlbäck durch eine Variante der APTT erfolgen (PCT/SE92/00310; WO 93710261), indem einmal die Gerinnung einer plättchenfreien Plasmaprobe einmal durch Calciumchlorid ohne Zusatz von aktiviertem Protein C (APC) und einmal durch Calciumchlorid mit Zusatz von APC ausgelöst wird. Die im Testgemisch entstandene Thrombinmenge wird entweder über die Umsetzung des natürlichen Substrats Fibrinogen zu einem Gerinnsel (Gerinnungszeit) oder über die Freisetzung eines Chromophors aus einem chromogenen Substrat photometrisch bestimmt. Das Vorliegen der Faktor V-Mutation macht sich dadurch bemerkbar, dass durch APC nur eine geringe Verlängerung der Gerinnungszeit erfolgt, während APC die APTT von Normalplasma stark verlängert. Durch Division der Gerinnungszeit der Probe mit APC durch die Gerinnungszeit der Probe ohne APC ergibt sich eine Verhältniszahl (Ratio) von diagnostischer Bedeutung. Eine Ratio über 2.0 wird bei Gesunden gefunden, eine Ratio zwischen 1.3-2.0 bei heterozygoten und eine Ratio unter 1.3 bei homozygoten Defektträgem. Da jedoch dieses Testsystem auf der Aktivierung der Gerinnung in Anwesenheit von Calciumionen beruht, können quantitative und qualitative Anomalien an calciumabhängigen Plasmagerinnungsfaktoren (FII,VII,VIII,IX,X,) das Resultat verfälschen. Mangel oder Dysfunktion von Protein S kann falsch positive und das Vorliegen von Antiphospholipid-Antikörpern (Lupus Antikoagulans) falsch negative Werte liefern. Die Anwesenheit von Plättchen in einer unsorgfältig zubereiteten Plasmaprobe kann das Resultat beeinflussen und schliesslich kann eine Therapie mit oralen Antikoagulantien oder Heparin das Testergebnis einer Plasmaprobe beeinflussen.

Im Anschluss an die Arbeiten von Dahlbäck konzentrierte sich die Forschung auf Verbesserungen oder Modifikationen des ursprünglichen Testsystems. So wird z.B. für eine spezifischere funktionelle Bestimmung der APC-Resistenz empfohlen, die zu untersuchende Probe vor Einsatz im Testverfahren mit FV-Mangelplasma im Verhältnis 20:80 zu mischen (Behringwerke EP 0711838 A1). Das eingesetzte FV-Mangelplasma sollte eine normale Konzentration an Faktor VIII enthalten, da sonst eine Verfälschung der Ergebnisse durch zu hohe oder zu niedrige Konzentrationen an FVIII in der Patientenprobe auftritt. Mit diesem Verfahren können Störungen durch Anomalien calciumabhängiger Plasmagerinnungsfaktoren vermindert werden (Witt I., Kraus M. APC-Resistenz: Klinik, Pathophysiologie und Diagnostik. Hämostaseologie, 1996; 16:60-67).

Der störende Einfluss von Heparin kann durch Zusatz eines Heparin-Antagonisten wie zum Beispiel Hexadimethrinbromid (Polybrene) vermindert werden, wobei aber nur die Verfälschung der Gerinnungszeit in Anwesenheit von APC korrigiert wird. Die APTT ohne Zusatz von APC bleibt hingegen verlängert, so dass in diesem Fall die APC-Ratio nicht zur Auswertung herangezogen werden kann. Zudem ist die Anwesenheit von Lupus Antikoagulans, die auch eine APTT-Verlängerung hervorruft, nicht mehr erkennbar. Daher kann in diesem Falle nicht auf einen zusätzlichen Test auf Lupus Antikoagulans verzichtet werden. Die Berechnung der APC-Ratio darf nicht vorgenommen werden, wenn eine abnorme APTT von mehr als 50% besteht.

Exner (PCT/AU95/00474; WO 96/04560) beschreibt Modifikationen des Dahlbäck-Patents. Dabei werden durch den Einsatz von Schlangengiften die endogenen Faktoren V und X aktiviert, wodurch eine erhöhte Sensitivität erreicht werden soll. Da auch dieses Testprinzip die Anwesenheit von Calciumionen erfordert, können auch mit dieser Testvariante nicht alle APC-resistenten Plasmen identifiziert und von den Normalplasmen unterschieden werden.

Die nach dem Stand der Technik zur Verfügung stehenden, im recalcifizierten Reaktionsgemisch arbeitenden, funktionellen Tests erlauben aus den oben dargelegten Gründen immer noch nicht mit 100%-iger Sicherheit eine APC-Resistenz zu diagnostizieren. Immer wieder liegen Plasmaproben im Grenzbereich, d.h. die Differenzierung zwischen Normalplasmen und Plasmen von Patienten mit heterozygot erworbener oder vererbter FV-Mutation und die Differenzierung zwischen heterozygoten und homozygoten Mutationsträgern ist oft nicht möglich. Eine definitive Abklärung ist daher nur durch Einsatz der wirtschaftlich, technisch und zeitlich aufwendigen Genomanalyse mittels PCR (Polymerase chain reaction) möglich.

Es wurde nun überraschenderweise gefunden, dass die FV-Abhängigkeit von Prothrombinaktivatoren aus bestimmten Schlangengiften, die in der Literatur als calcium- phospholipid- und FV-abhängig beschrieben werden, in Abwesenheit von Calciumionen stärker als in Anwesenheit von Calciumionen ist. Es wurde ferner gefunden, dass die stimulierende Cofaktorwirkung von Faktor Va besonders deutlich in Erscheinung tritt, wenn statt Calciumionen sogar ein Calcium-Komplexbildner, wie z.B. der Chelator Ethylendiamintetraessigsäure (EDTA), dem Testgemisch beigefügt wird (Beispiel 1). Es wurde schliesslich gefunden, dass sich der besagte Schlangengiftaktivator in Abwesenheit von Calciumionen im Testgemisch hervorragend zum spezifischen, diagnostischen Nachweis von erworbener oder angeborener APC-Resistenz eignet.

Das Verfahren besteht grundsätzlich darin, dass man eine Plasmaprobe mit einem Protein C-Aktivator und/oder APC inkubiert, die Gerinnung durch Zugabe eines calciumunabhängigen, jedoch FV-abhängigen Prothrombinaktivators, jedoch ohne Zusatz von Calciumionen auslöst und die Gerinnungszeit misst und mit der Gerinnungszeit eines Referenzplasmas vergleicht. Die Gerinnungszeit wird bei Normalplasma verlängert, nicht aber bei Plasma mit APC Resistenz, so dass aus dem Vergleich der Gerinnungszeit der Plasmaprobe mit der Gerinnungszeit des Referenzplasmas leicht auf die Anwesenheit resp. Abwesenheit einer APC-Resistenz geschlossen werden kann.

Das Referenzplasma ohne APC-Resistenz kann dabei aus einer Anzahl von normalen Plasmen, einer Mischung von normalen und/oder heterozygoten und/oder homozygoten Plasmen oder einem Einzelplasma bestehen (= Normalplasma). Der Vergleich der Gerinnungszeiten der Plasmaprobe mit derjenigen des Referenzplasmas kann mit oder auch ohne Zusatz eines Protein C-Aktivators resp. APC zum Referenzplasma erfolgen.

Ferner kann als Referenzplasma auch ein Teil der zu untersuchenden Plasmaprobe verwendet werden.

Die Geräte zur Messung der Gerinnungszeit können mit einem Referenzplasma so eingestellt werden, dass die Gerinnungszeit des Referenzplasmas z.B. ca. 80 - 140 Sekunden beträgt. Das Verhältnis zwischen mutiertem und normalem Faktor V im Referenzplasma kann dabei z.B. zwischen 20:80 und 80:20 sein.

Die Einstellung der Geräte mit Referenzplasmen kann z.B. einmal monatlich oder auch jeweils parallel zu den Messungen der Plasmaproben erfolgen.

Es ist jedoch nicht erforderlich, die Geräte zur Messung der Gerinnungszeit einzustellen. Es reicht in der Regel vollkommen aus, die Gerinnungszeit einer Plasmaprobe zu messen und mit den Erfahrungswerten von Referenzplasmen, die z.B. den Testkits in Form von tabellarischen Werten beigelegt werden, zu vergleichen. Aus den verglichenen Werten kann dann auf homozygotes, heterozygotes oder normales Blutplasma geschlossen werden. Dies ist verglichen mit den gemäss dem Stand der Technik notwendigen Messverfahren eine erhebliche Vereinfachung.

Falls ein Teil der zu untersuchenden Plasmaprobe gleichzeitig auch als Referenzplasma dient, kann man a) einen Teil einer Plasmaprobe mit einem Protein C-Aktivator und/oder APC inkubieren, die Gerinnung durch Zugabe eines calciumunabhängigen Prothrombinaktivators ohne Zusatz von Ca²⁺-lonen im Testsystem auslösen, b) bei einem andern Teil der Plasmaprobe ohne Zugabe eines Protein C-Aktivators und/oder APC die Gerinnung durch Zugabe eines calciumunabhängigen Prothrombinaktivators ohne Zusatz von Ca²⁺-lonen im Testsystem auslösen, und c) die Gerinnungszeit messen und und dem Vergleich der beiden Gerinnungszeiten oder aus der Grösse des Quotienten der beiden Gerinnungszeiten aus den Teilen der jeweiligen Probe unter a) und b) hiervor die Störung im Protein C-System nachweisen.

Die Empfindlichkeit des Verfahrens wird durch Zusatz eines Calcium-Komplexbildners erheblich erhöht werden und dessen Spezifität für mutierten FV kann durch Verdünnen der Plasmaprobe mit FV-freiem Plasma gesteigert werden.

Auf die Durchführung je eines Tests mit und ohne APC-Zusatz und auf die Berechnung einer Ratio kann verzichtet werden, wenn das erfindungsgemässe Verfahren unter standardisierten Bedingungen bezüglich Reagenzien, Hilfsstoffe und Apparaturen durchgeführt wird. In diesem Falle kann aus der Gerinnungszeit bezw. der Substratspaltung direkt auf die Qualität des FV in der Plasmaprobe geschlossen werden.

Der Vorteil dieses Verfahrens besteht darin, dass Ca²⁺-abhängige und zur Prothrombinaktivierung führende Konkurrenz- oder Störreaktionen, erhöhte oder erniedrigte Konzentration an Faktor VIII, spezifische, inhibitorische Antikörper gegen bestimmte Plasmabestandteile, wie z.B. Lupus Antikoagulans, Anwesenheit von Plättchen, Plasmen von oral antikoagulierten und heparinisierten Patienten durch die Abwesenheit von Calciumionen unterdrückt werden.

Ein besonderer Vorteil des erfindungsgemässen Verfahrens besteht ferner in der spezifischen funktionellen Erfassung von strukturellen Veränderungen von FV und insbesondere der häufigen Mutation FV:Q⁵⁰⁶ (FV Leiden). Dieser Anteil kann im Normalfall sowohl durch Bestimmung der Plasmagerinnungszeit in Gegenwart von APC (Beispiele 3 und 5) als auch in Gegenwart eines geeigneten Protein C-Aktivators (Beispiele 2 und 4) bei Prothrombinaktivierung in Abwesenheit von Calciumionen und gegebenenfalls Anwesenheit von Chelatoren wie EDTA und Vergleich dieser Plasmagerinnungszeit mit derjenigen eines Referenzplasmas erhalten werden. Als geeigneter Protein C-Aktivator kommt z.B. das von der Firma Pentapharm AG kommerziell erhältliche Protac® (Kurt F. Stocker und Lars G. Svendsen, EP 0 203 509 81) in Frage. Dabei ist die Spezifität der beiden Methoden (APC resp. Protein C-Aktivator) so gross, dass homozygote Träger von APC-resistentem Faktor V, heterozygote Träger von APC-resistentem FV und Normalpopulationen in sehr guter Approximation unterschieden werden können. Ferner lassen die erhaltenen Werte auch zu, dass man feststellen kann, ob heterozygote Träger mehr oder weniger APC-resistenten FV enthalten.

Das erfindungsgemässe Verfahren lässt sich sehr gut auf verschiedene Techniken der Blutgerinnungsanalyse adaptieren. So können zum Nachweis der zu bestimmenden Werte auch chromogene, fluorogene, amperogene Substrate oder andere Bestimmungsmethoden nach dem Stand der Technik verwendet werden. Dementsprechend kann die Zusammensetzung des Testgemisches durch Veränderung der Natur oder Menge der Chelatoren, durch gezielte Einstellung des pH-Werts, durch die Zugabe bestimmter Inhibitoren des Gerinnungssystems (inkl. PC-System) den methodischen und technischen Erfordernissen angepasst werden.

Als Protein C-Aktivatoren lassen sich nebst dem handelsüblichen Protac® grundsätzlich auch Protein C-Aktivatoren oder gegebenenfalls gereinigte Fraktionen aus Giften der Schlangen Agkistrodon contortrix und ihrer Unterarten, Agkistrodon piscivorus und ihrer Unterarten, Agkistrodon bilineatus und ihrer Unterarten oder Agkistrodon halys und ihrer Unterarten verwenden.

Da mit dem Verfahren grundsätzlich nicht nur Anomalien von Faktor V sondern auch von Protein C oder Protein S erfasst werden können, kann es vorteilhaft sein, entsprechende Mangelplasmen, z. B. FV-, Protein C - oder Protein S-Mangelplasma, zuzusetzen, wobei das Testverfahren nicht empfindlich auf die vorhandenen Mengen an Mangelplasma reagiert, so dass man - ohne die erfassten Werte wesentlich zu verschieben - geringe Mengen von z.B. 1% bis zu grossen Mengen von z.B. 99% Mangelplasma zugeben kann.

Vorteilhafterweise werden ≥ 50% Mangelplasma benutzt.

Ein Zusatz von Phospholipiden ist für die Durchführung des erfindungsgemässen Verfahrens nicht erforderlich. Da jedoch Plasma, abhängig vom Verlauf seiner Zubereitung, unterschiedliche Phospholipidspuren enthält, könnte ein Phospholipidzusatz die Schwankungsbreite der Testresultate einschränken.

Als erfindungsgemäss verwendete calciumunabhängige Prothrombinaktivatoren können FV-abhängige Schlangengiftenzyme sowohl in Form von Rohgiften als auch gereinigten Giftfraktionen verwendet werden. Calciumunabhängig sind die Prothrombinaktivatoren in bezug auf ihre Fähigkeit, ihre erfindungsgemässe Funktion auch ohne Zusatz von Calcium wirkungsvoll ausüben zu können. Die Prothrombinaktivator-Zubereitungen können im Hinblick auf die praktische Anwendung mit an sich bekannten stabilisierenden Zusätzen versehen werden. Geeignete Schlangengifte oder gereinigte Schlangengiftfraktionen zur Zubereitung von erfindungsgemässen Prothrombinaktivator-Präparaten sind Gifte mit einer dominierenden FV-abhängigen prothrombinaktivierenden Wirkung, die sich auch ohne Calciumzusatz entfaltet. Erfindungsgemäss werden Schlangengifte, vorzugsweise aus der Elapidengattung Notechis, Tropidechis, Cryptophys, Hoplocephalus und Pseudechis, wie z.B. von Notechis scutatus scutatus, Notechis ater niger, Notechis ater humphreysi, Notechis ater serventyi, Notechis flinders, Notechis occidentalis, Tropidechis carinatus, Cryptophis nigrescens, Hoplocephalus stephensii und Pseudechis porphyriacus verwendet (Beispiel 6). Nebst Prothrombinaktivatoren aus Schlangengift können grundsätzlich auch aus Mikroorganismen mit natürlichem oder rekombiniertem Genom gewonnene Aktivatoren verwendet werden.

Der erfindungsgemäss speziell wegen seiner grossen Verbreitung bevorzugte Chelator ist EDTA, aber die Untersuchung verschiedener Ca²⁺-Chelatoren und Calciumfällungsmittel hat gezeigt, dass auch andere, strukturell von EDTA abweichende Substanzen, wie z.B. Citrat, Oxalat in Frage kommen (Beispiel 7). Zu den Chelatoren sind u. a. auch EGTA (Ethylenbis-(oxyethylennitrilo)-tetraessigsäure), Desferoxamin, Tetracycline, BAPTA (1,2-Bis-(2-aminophenoxy)-ethan-N,N,N',N'-tetraessigsäure) und deren Salze und Quin-2 (2-[(2-Amino-5-methylphenoxy)-methyl]-6-meihoxy-8-aminochinolin-N,N,N',N'-tetraessigsäure Tetrakaliumsalz zu zählen. Auch andere Methoden können zur Entfernung des überschüssigen Calciums verwendet werden, wie z.B. Fällungen mit Sulfat oder Carbonat, Adsorption u.a. oder Verfahren, welche den EDTA-Effekt auf die FV abhängigen Prothrombinaktivatoren ausüben.

Für die Erfassung der FV-abhängigen, APC-empfindlichen Prothrombinaktivierung kann die Bestimmung der Plasmagerinnungszeit. manuell oder automatisch, mechanisch, elektromagnetisch oder photometrisch erfolgen. In einem erfindungsgemässen Testgemisch generiertes Thrombin kann auch photometrisch, fluorimetrisch oder amperometrisch unter Verwendung geeigneter synthetischer, chromogener bezw. fluorogener oder amperogener Substrate bestimmt werden. (Beispiel 8). Für ein Übersichtsreferat über synthetische Substrate in der Hämostaseologie siehe Witt I. Test Systems with synthetic peptide substrates in haemostaseology, Eur. J. Clin. Chem. Clin. Biochem., 1991, 29: 355-374. Als chromogenes Substrat geeignet ist z.B. das von der Firma Pentapharm AG kommerziell erhältliche Tos-Gly-Pro-Arg-pNA-AcOH (Pefachrom TH).

Erfindungsgemäss können Störeinflüsse durch heparinisierte Plasmen mit einem Heparin-Antagonisten, wie z.B. Polybrene, Protaminsalze oder heparinspaltende Enzyme vermieden werden. Mit, aber auch ohne Heparin-Antagonisten kann z.B. entgegen dem Stand der Technik zwischen homozygotem FV-Defekt, heterozygotem FV-Defekt und Normalplasmen deutlich unterschieden werden (Beispiel 9).

Die Inkubationsphase im erfindungsgemässen Verfahren kann wesentlich verkürzt werden, indem man dem Testgemisch einen Faktor V-Aktivator beifügt. Als Faktor V -Aktivator kann z.B. RVV-V aus Vipera russelli oder z.B. auch Faktor V-Aktivatoren aus Giften der Schlangen Bothrops atrox, Bothrops jararaca, Naja n. oxiana, Echis carinatus, Echis multisquamatus, Vipera ursini, Vipera lebetina, Haemachatus haemachatus, Naja m. Mossambica, Naja nivea, Naja nigricollis, Naja h. haje, Naja n. kaouthia, Naja melanoleuca, Pseudechis australis, Pseudonaja t. textilis, Notechis ater, Oxyuranus scutellatus oder der Raupe Lonomia achelous verwendet werden.

Die Bestimmung der APC-Resistenz nach der Entnahme und der den Umständen angepassten Aufbereitung und Stabilisierung der Probe läuft bevorzugt in zwei in der Praxis allerdings kaum trennbaren Schritten ab. Die zwei Schritte können als Inkubationsphase und als Prothrombinaktivierungsphase bezeichnet werden. Als Proben eignen sich dabei z.B. citrat-stabilisierte Plasmaproben. Man kann aber auch Stabilisatoren wie z. B. Oxalat oder EDTA verwenden. Während der Inkubationsphase erfolgt, vorzugsweise in Anwesenheit von FV-Mangelplasma, die Aktivierung des FV aus der Probe, welche in an sich bekannter Weise z.B. mit RVV-V (aus Vipera russelli) erreicht werden kann. Diese Aktivierungsreaktion mit oder ohne APC oder einem Protein C-Aktivator fällt in die Inkubationsphase, wobei die benötigten Reagenzien bevorzugt zu Beginn der Inkubationsphase beigefügt werden. Die Prothrombinaktivierungsphase beginnt mit der Zugabe des FV-abhängigen Schlangengiftes resp. der entsprechenden gereinigten Fraktion des Schlangengifts. Durch den Zusatz eines Chelators (z.B. EDTA) zur Probe kann die Spezifität gesteigert werden. Ein solcher Chelator wird typischerweise, aber nicht unbedingt zwingend, erst zusammen mit dem FV-abhängigen Schlangengiftenzym beigefügt.

Anzumerken ist, dass das erfindungsgemässe Verfahren auch Variationen mit überlappender, schrittweiser oder gar kontinuierlicher Zugabe einzelner oder mehrerer der erwähnten Komponenten nicht ausschliesst. Die Dauer der lnkubationsphase hängt u.a. von der Fragestellung und der Natur der verwendeten Aktivierung des APC-Systems ab. Sie liegt typischerweise im Bereich zwischen 1 und 40 Minuten, vorzugsweise aber unter 30 Minuten.

Erfindungsgemässe Kits, die man zum Nachweis von Störungen im Protein C-System verwenden kann, enthalten APC oder Protein C-Aktivatoren, wie z.B. Protac, einen Prothrombinaktivator und gegebenenfalls einen Faktor V-Aktivator, wie z.B. RW-V. Ferner können diesen Kits auch Phospholipide, Faktor V-Mangelplasma, ein Ca-Komplexbildner, wie z.B. EDTA, und je nach benütztem Nachweisverfahren ein oder mehrere Referenzplasmen, und ein Heparin-Antagonist beigefügt werden.

Im folgenden wird die Erfindung anhand von Beispielen näher erläutert. Dabei werden als Abkürzungen verwendet:
- APC:: Aktiviertes Protein C
- RW-V:: FV Aktivator aus Vipera russelli
- Protac®:: Protein C Aktivator aus Agkistrodon contortrix contortrix

Die Art der Stabilisierung der verwendeten Plasmaproben der Versuchspersonen ist dabei ohne Bedeutung, weil nicht nur mit citrat-stabilisierten Proben repräsentative Resultate erhältlich sind. Die Plasmaproben wurden alle auf APC-Resistenz mit dem direkten Nachweis der Mutation auf DNA-Ebene untersucht.

### Bsp. 1: FV-Abhängigkeit des Prothrombinaktivators aus Notechis scutatus scutatus-Gift

Die Gerinnungszeit wurde mit einem Koagulometer KC4 (Amelung, Lemgo, Deutschland) bestimmt. 40 µl FV Mangelplasma, 10 µl Plasmaprobe (+ FV) oder 50 µl FV-Mangelplasma (- FV) wurden mit 50 µl 50 mM Hepes, pH 7.5 1 Minute bei 37°C inkubiert. Die Gerinnung wurde durch die Zugabe von 50 µl 5 µg/ ml Prothrombinaktivator aus Notechis scutatus scutatus-Gift in 25 mM CaCl₂, 5 µg/ ml Prothrombinaktivator ohne Zusätze oder in 10-40 mM EDTA (Tabelle 2), ausgelöst. Alle Reagenzien wurden in 50 mM Hepes, pH 7.5 gelöst. Die Gerinnungszeit wird in Anwesenheit und Abwesenheit von FV bestimmt. Aus der Gerinnungszeit mit FV und ohne FV wird ein Verhältnis (Ratio) gebildet.

Die erhaltenen Resultate zeigen wie die Zugabe von Calciumionen die FV-Abhängigkeit des Testsystems verschlechtert. Ohne Zugabe von Calciumionen oder mit Zusatz von EDTA konnte die FV-Abhängigkeit des Testsystemes erheblich verstärkt werden.

**Tabelle 2**

| Zusätze | Prothrombinaktivator µg/ml | Gerinnungszeit | | Quotient + FV/-FV |
|---|---|---|---|---|
| | | + FV | - FV | |
| 25 mM CaCl₂ | 0.5 | 51.6 | 106.0 | 2.05 |
| ohne Zusätze | 5 | 46.3 | 141.3 | 3.05 |
| 10 mM EDTA | 5 | 39.2 | 196.3 | 5.00 |
| 20 mM EDTA | 5 | 48.0 | 262.6 | 5.48 |
| 30 mM EDTA | 5 | 59.3 | 334.5 | 5.64 |
| 40 mM EDTA | 5 | 72.0 | 472.3 | 6.56 |

### Bsp. 2: Bestimmen der APC-Resistenz mit Protac®

Die Gerinnungszeit wurde mit einem Koagulometer KC4 micro (Amelung, Lemgo, Deutschland) bestimmt. 40 µl FV Mangelplasma, 10 µl Plasmaprobe und 50 µl 2 U/ml Protac®, 1 U/ml RVV-V und 0.1 mg/ml Kephalin wurden 20 Minuten bei 37° C inkubiert. Die Gerinnung wurde durch die Zugabe von 50 µl 5 µg/ml Prothrombinaktivator aus Notechis scutatus scutatus-Gift in 15 mM EDTA ausgelöst. Alle Reagenzien wurden in 50 mM Hepes, pH 7.5 gelöst. Die Gerinnungszeit einer Plasmaprobe wird mit der Gerinnungszeit einer Plasmaprobe aus einem APC-resistenzfreien Plasmapool resp. mit APC-resistenzfreien Normalplasmen verglichen. Aus den beiden Gerinnungszeiten mit Protac® von Plasmaprobe zu Plasmapool wird ein Verhältnis (Quotient) gebildet.

Die erhaltenen Resultate zeigen, wie der Quotient vom Plasmapool über Plasmen mit heterozygotem FV-Defekt zu Plasmen mit homozygotem FV-Defekt in einem Masse abnimmt, dass nicht nur zwischen Normalplasmen und solchen mit FV-Defekten, sondern auch innerhalb der defekten Plasmen zwischen heterozygoten und homozygoten FV-Defekten unterschieden werden kann.

**Tabelle 3**

| Plasmaprobe | Gerinnungszeit + Protac® [s] | Gerinnungszeit Plasmaprobe/ Gerinnungszeit Plasmapool |
|---|---|---|
| 1. Plasmapool ohne APC-Resistenz | 97.4 | 1.00 |
| 2. Homozygoter FV Defekt | 44.7 | 0.46 |
| 3. Heterozygoter FV Defekt | 59.9 | 0.62 |
| 4. Heterozygoter FV Defekt | 66.0 | 0.68 |
| 5. Heterozygoter FV Defekt | 61.4 | 0.63 |
| 6. Heterozygoter FV Defekt | 60.3 | 0.62 |
| 7. Heterozygoter FV Defekt | 67.2 | 0.69 |
| 8. Heterozygoter FV Defekt | 61.9 | 0.64 |
| 9. Heterozygoter FV Defekt | 69.7 | 0.72 |
| 10. Normal | 96.5 | 0.99 |
| 11. Normal | 82.3 | 0.85 |
| 12. Normal | 101.4 | 1.04 |
| 13. Normal | 93.6 | 0.96 |
| 14. Normal | 103.1 | 1.06 |
| 15. Normal | 111.1 | 1.14 |
| 16. Normal | 97.6 | 1.00 |
| 17.Normal | 94.7 | 0.97 |
| 18. Normal | 95.3 | 0.98 |
| 19. Normal | 98.3 | 1.01 |

### Bsp. 3: Bestimmen der APC-Resistenz mit APC

Die Gerinnungszeit wurde mit einem Koagulometer KC4 micro (Amelung, Lemgo, Deutschland) bestimmt. 40 µl FV Mangelplasma, 10 µl Plasmaprobe und 50 µl 10 µg/ml APC, 10 U/ml RVV-V und 0.1 mg/ml Kephalin wurden 8 Minuten bei 37° C inkubiert. Die Gerinnung wurde durch die Zugabe von 50 µl 5 µg/ml Prothrombinaktivator aus Notechis scutatus scutatus-Gift in 15 mM EDTA ausgelöst. Alle Reagenzien wurden in 50 mM Hepes, pH 7.5 gelöst. Die Gerinnungszeit einer Plasmaprobe wird mit der Gerinnungszeit einer Plasmaprobe aus einem APC-resistenzfreien Plamapool verglichen. Aus der Gerinnungszeit der Plasmaprobe mit APC und der ermittelten Gerinnungszeit der Plasmen im Plasmapool wird ein Verhältnis gebildet (Tabelle 4).

Die erhaltenen Resultate zeigen, dass durch die exogene Zugabe von APC die Gerinnungszeit des Plasmapools resp. der Normalplasmen verlängert wird, während die Gerinnungszeit der Plasmen mit heterozygotem und insbesondere mit homozygotem FV-Defekt signifikant und in dem Masse kürzer ausfällt, dass nicht nur zwischen gesunden Plasmen und solchen mit FV-Defekt, sondern auch zwischen heterozygoten und homozygoten FV-Defekten unterschieden werden kann.

**Tabelle 4**

| Plasmaprobe | Gerinnungszeit + APC [s] | Gerinnungszeit Plasmaprobe/ Gerinnungszeit Plasmapool |
|---|---|---|
| 1. Plasmapool ohne APC-Resistenz | 90.0 | 1.00 |
| 2. Homozygoter FV Defekt | 54.8 | 0.60 |
| 3. Heterozygoter FV Defekt | 59.9 | 0.67 |
| 4. Heterozygoter FV Defekt | 66.0 | 0.73 |
| 5. Heterozygoter FV Defekt | 61.4 | 0.68 |
| 6. Heterozygoter FV Defekt | 58.9 | 0.65 |
| 7. Heterozygoter FV Defekt | 60.3 | 0.67 |
| 8. Heterozygoter FV Defekt | 60.9 | 0.68 |
| 9. Heterozygoter FV Defekt | 67.3 | 0.75 |
| 10.Normal | 96.5 | 1.07 |
| 11. Normal | 77.5 | 0.86 |
| 12.Normal | 79.8 | 0.88 |
| 13.Normal | 82.3 | 0.91 |
| 14.Normal | 101.4 | 1.13 |
| 15.Normal | 76.6 | 0.85 |
| 16.Normal | 93.6 | 1.04 |
| 17. Normal | 103.1 | 1.15 |
| 18.Normal | 79.6 | 0.88 |
| 19.Normal | 111.1 | 1.23 |

### Bsp. 4: Bestimmen der APC-Resistenz mit Protac®

Die Gerinnungszeit wurde mit einem Koagulometer KC4 (Amelung, Lemgo, Deutschland) bestimmt. 40 µl FV Mangelplasma, 10 µl Plasmaprobe und 50 µl 2 U/ml Protac®, 1 U/ml RVV-V und 0.1 mg/ml Kephalin wurden 20 Minuten bei 37° C inkubiert Die Gerinnung wurde durch die Zugabe von 50 µl 5 µg/ ml Prothrombinaktivator aus Notechis scutatus scutatus-Gift in 25 mM CaCl₂ (Tabelle 5 A), ohne Zusätze (Tabelle 5 B) oder in 15 mM EDTA (Tabelle 5 C) ausgelöst. Alle Reagenzien wurden in 50 mM Hepes, pH 7.5 gelöst. Die Gerinnungszeit wird in Anwesenheit und Abwesenheit von Protac® bestimmt. Aus der Gerinnungszeit mit Protac® und ohne Protac® wird ein Verhältnis (Ratio) gebildet.

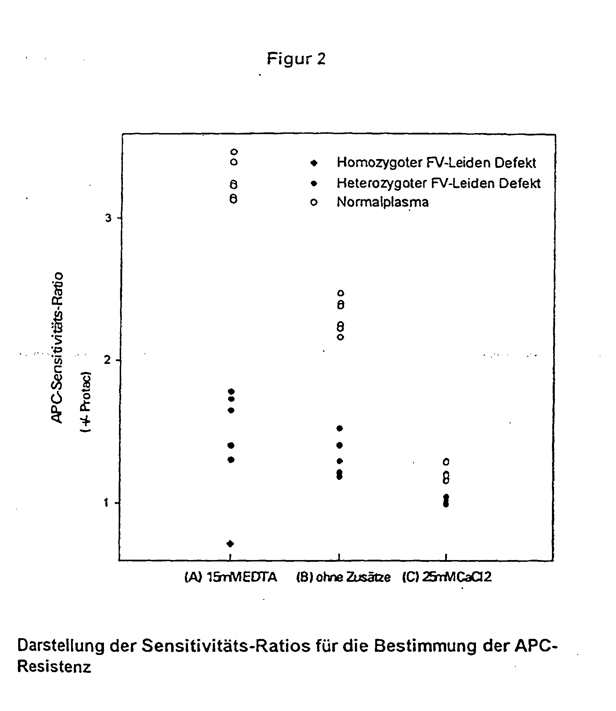

Die erhaltenen Resultate zeigen wie die Ratio durch die Zugabe von Calciumionen bei normalen Plasmaproben stark verringert wird und zwischen den Normalen Plasmaproben und den Plasmaproben mit heterozygotem FV-Defekt kaum unterschieden werden kann. Durch die Zugabe von EDTA wird die Sensitivität zwischen normalen, heterozygoten und homozygoten Plasmaproben überraschenderweise stark erhöht (Figur 2).

**Tabelle 5**

| **A** | | | |
|---|---|---|---|
| Plasmaprobe | Gerinnungszeit + Protac® [s] | Gerinnungszeit - Protac® [s] | **Ratio +/- Protac®** |
| 1. Heterozygoter FV Defekt | 28.3 | 27.9 | **1.01** |
| 2. Heterozygoter FV Defekt | 31.9 | 31.5 | **1.01** |
| 3. Heterozygoter FV Defekt | 26.0 | 26.3 | **0.99** |
| 4. Heterozygoter FV Defekt | 26.1 | 25.9 | **1.01** |
| 5. Heterozygoter FV Defekt | 43.5 | 41.7 | **1.04** |
| 6. Normal | 31.9 | 27.4 | **1.16** |
| 7. Normal | 34.1 | 29.7 | **1.15** |
| 8. Normal | 37.0 | 31.2 | **1.19** |
| 9. Normal | 37.8 | 31.4 | **1.20** |
| 10.Normal | 37.5 | 31.4 | **1.19** |
| 11.Normal | 46.3 | 35.8 | **1.29** |
| 12. Normal | 50.9 | 39.9 | **1.28** |

| **B** | | | |
|---|---|---|---|
| Plasmaprobe | Gerinnungszeit + Protac®[s] | Gerinnungszeit - Protac®[s] | **Ratio +/- Protac®** |
| 1. Heterozygoter FV Defekt | 65.5 | 50.9 | **1.29** |
| 2. Heterozygoter FV Defekt | 89.5 | 63.8 | **1.40** |
| 3. Heterozygoter FV Defekt | 55.0 | 46.6 | **1.18** |
| 4. Heterozygoter FV Defekt | 53.5 | 44.2 | **1.21** |
| 5. Heterozygoter FV Defekt | 125.7 | 82.8 | **1.52** |
| 6. Normal | 103.1 | 46.0 | **2.24** |
| 7. Normal | 94.6 | 42.1 | **2.25** |
| 8. Normal | 114.4 | 47.7 | **2.40** |
| 9. Normal | 114.2 | 47.9 | **2.38** |
| 10.Normal | 101.4 | 47.0 | **2.16** |
| 11.Normal | 134.4 | 54.3 | **2.47** |
| 12.Normal | 139.0 | 62.6 | **2.22** |

| **C** | | | |
|---|---|---|---|
| Plasmaprobe | Gerinnungszeit + Protac® [s] | Gerinnungszeit - Protac®[s] | **Ratio +/- Protac®** |
| 1: Homozygoter FV Defekt | 44.7 | 53.6 | **0.83** |
| 2. Homozygoter FV Defekt | 57.6 | 79.9 | **0.72** |
| 3. Heterozygoter FV Defekt | 135.6 | 82.1 | **1.65** |
| 4. Heterozygoter FV Defekt | 205.4 | 119.0 | **1.73** |
| 5. Heterozygoter FV Defekt | 101.2 | 77.9 | **1.30** |
| 6. Heterozygoter FV Defekt | 96.9 | 69.1 | **1.40** |
| 7. Heterozygoter FV Defekt | 332.9 | 186.6 | **1.78** |
| 8. Normal | 258.8 | 76.1 | **3.40** |
| 9. Normal | 196.8 | 62.5 | **3.15** |
| 10.Normal | 250.1 | 77.0 | **3.25** |
| 11.Normal | 262.5 | 81.2 | **3.23** |
| 12.Normal | 230.1 | 73.1 | **3.15** |
| 13.Normal | 307.5 | 88.5 | **3.47** |
| 14.Normal | 342.8 | 109.5 | **3.13** |

### Bsp. 5: Bestimmen der APC-Resistenz mit APC

Die Gerinnungszeit wurde mit einem Koagulometer KC4 micro (Amelung, Lemgo, Deutschland) bestimmt. 40 µl FV Mangelplasma, 10 µl Plasmaprobe und 50 µl 10 µg/ml APC, 10 U/ml RVV-V und 0.1 mg/ml Kephalin wurden 8 Minuten bei 37° C inkubiert. Die Gerinnung wurde durch die Zugabe von 50 µl 5 µg/ ml Prothrombinaktivator aus Notechis scutatus scutatus-Gift in 15 mM EDTA ausgelöst. Alle Reagenzien wurden in 50 mM Hepes, pH 7.5 gelöst. Die Gerinnungszeit wird in Anwesenheit und Abwesenheit von APC bestimmt. Aus der Gerinnungszeit mit APC und ohne APC wird ein Verhältnis (Ratio) gebildet (Tabelle 6).

Die erhaltenen Resultate zeigen, dass durch die exogene Zugabe von APC die Inkubation verkürzt werden kann, ohne einen Sensitivitätsverlustes des Testsystems zu erreichen. Die Normalplasmen können in jedem Fall von den Heterozygoten getrennt werden. Auch in diesem Beispiel wird wieder auf die Zugabe von Calciumionen verzichtet.

**Tabelle 6**

| Plasmaprobe | Gerinnungszeit + APC [s] | Gerinnungszeit - APC [s] | **Ratio +/- APC** |
|---|---|---|---|
| 1. Normal | 126.0 | 43.0 | **2.93** |
| 2. Heterozygoter FV Defekt | 64.7 | 45.0 | **1.44** |
| 3. Heterozygoter FV Defekt | 68.8 | 47.0 | **1.46** |
| 4. Heterozygoter FV Defekt | 67.3 | 45.3 | **1.49** |

### Bsp. 6. FV abhängige oder unabhängige Prothrombinaktivatoren aus Schlangengiften

Die Gerinnungszeit wurde mit einem Koagulometer KC4 micro (Amelung, Lemgo, Deutschland) bestimmt. 40 µl FV Mangelplasma, 10 µl Plasmaprobe und 50 µl 2 U/ml Protac®, 1 U/ml RVV-V und 0.1 mg/ml Kephalin wurden 20 Minuten bei 37° C inkubiert. Die Gerinnung wurde durch die Zugabe von 50 µl 5-50 µg/ ml ungereinigtes Schlangengift von Schlangen mit FV-abhängigen oder FVunabhänigen Prothrombinaktivatoren in 15 mM EDTA oder 5 µg/ml gereinigter Prothrombinaktivator in 15 mM EDTA ausgelöst. Alle Reagenzien wurden in 50 mM Hepes, pH 7.5 gelöst. Die Gerinnungszeit wird in Anwesenheit und Abwesenheit von Protac® bestimmt. Aus der Gerinnungszeit mit Protac® und ohne Protac® wird eine Ratio gebildet (Tabelle 5).

Die Resultate zeigen, wie gereinigte FV abhängige Prothrombinaktivatoren oder Schlangenrohgifte mit FV-abhängigen Prothrombinaktivatoren für die Bestimmung der APC-Resistenz verwendet werden können. FV-abhängige Prothrombinaktivatoren ergeben eine Ratio, mit deren Hilfe die zwischen den Plasmaproben mit APC-Resistenz und Normalplasma unterschieden werden kann.

**Tabelle 7**

| Normale Plasmaproben: | | | | |
|---|---|---|---|---|
| FV abhängige Schlangengifte oder gereinigte Prothrombinaktivatoren* | | Gerinnungszeit + Protac® [s] | Gerinnungszeit - Protac® [s] | **Ratio +/- Protac®** |
| 1. Notechis ater occidentatis | (25 µg/ml) | 166.7 | 70.8 | **2.35** |
| 2. Notechis ater niger | (25 µg/ml) | 305.2 | 105.7 | **2.89** |
| 3. Notechis ater humphreysi | (25 µg/ml) | 152.6 | 60.5 | **2.52** |
| 4. Notechis ater serventyi | (25 µg/ml) | 179.5 | 59.7 | **3.01** |
| 5. Pseudechis porphyriacus | (50 µg/ml) | 390.0 | 144.6 | **2.70** |
| 6. Hoplocephalus stephensii | (50 µg/ml) | 208.4 | 78.1 | **2.67** |
| 7. *Tropidechis carinatus | ( 5 µg/ml) | 135.6 | 55.2 | **2.46** |

| Heterozygoter FV Defekt: | | | | |
|---|---|---|---|---|
| FV abhängige Schlangengifte oder gereinigte Prothrombinaktivatoren* | | Gerinnungszeit + Protac® [s] | Gerinnungszeit - Protac® [s] | **Ratio +/- Protac®** |
| Notechis ater occidentalis | (25 µg/ml) | 122.8 | 80.3 | **1.53** |
| Notechis ater niger | (25 µg/ml) | 137.3 | 90.9 | **1.51** |
| Notechis ater humphreysi | (25 µg/ml) | 78.5 | 60.3 | **1.30** |
| Notechis ater serventyi | (25 µg/ml) | 79.8 | 61.3 | **1.30** |
| Pseudechis porphyriacus | (50 µg/ml) | 204.8 | 158.1 | **1.30** |
| Hoplocephalus stephensii | (50 µg/ml) | 114.6 | 82.4 | **1.39** |
| *Tropidechis carinatus | ( 5 µg/ml) | 94.9 | 65.4 | **1.45** |

| Normale Plasmaproben: | | | | |
|---|---|---|---|---|
| FV unabhängige Schlangengifte oder gereinigte Prothrombinaktivatoren* | | Gerinnungszeit + Protac® [s] | Gerinnungszeit - Protac® [s] | **Ratio +/- Protac®** |
| Akgistrodon rhodostoma (4) | (25 µg/ml) | 51.5 | 57.5 | **0.90** |
| Crotalus adamanteus (12) | (25 µg/ml) | 70.8 | 77.2 | **0.92** |
| Oxyuranus scutellatus (307) | (25 µg/ml) | 21.9 | 21.7 | **1.01** |
| Oxyuranus microlepidotus (337) | (25 µg/ml) | 19.0 | 19.3 | **0.98** |
| Pseudonaja textilis | (25 µg/ml) | 7.8 | 7.7 | **1.01** |
| | (5 µg/ml) | 21.0 | 21.4 | **0.98** |
| Bothrops neuwiedi | (25 µg/ml) | 157.8 | 160.9 | **0.98** |
| *Ecarin | (5 µg/ml) | > 600 | > 600 | **-** |
| *Oxyuranus scutellatus | (5 µg/ml) | 21.0 | 21.0 | **1.00** |
| *Textarin® | (5 µg/ml) | > 600 | > 600 | **-** |

| Heterozygoter FV Defekt: | | | | |
|---|---|---|---|---|
| FV unabhängige Schlangengifte oder gereinigte Prothrombinaktivatoren* | | Gerinnungszeit + Protac® [s] | Gerinnungszeit - Protac® [s] | **Ratio +/- Protac®** |
| Akgistrodon rhodostoma | (25 µg/ml) | 50.8 | 53.5 | **0.95** |
| Crotalus adamanteus | (25 µg/ml) | 65.2 | 74.6 | **0.87** |
| Oxyuranus scutellatus | (25 µg/ml) | 26.5 | 25.8 | **1.03** |
| Oxyuranus microlepidotus | (25 µg/ml) | 18.2 | 19.6 | **0.93** |
| Pseudonaja textilis | (5 µg/ml) | 21.2 | 21.6 | **0.98** |
| Bothrops neuwiedi | (25 µg/ml) | 155.9 | 154.5 | **1.01** |
| *Ecann | (5 µg/ml) | > 600 | > 600 | **-** |
| *Oxyuranus scutellatus | (5 µg/ml) | 21.0 | 20.8 | **1.01** |
| *Textarin® | (5 µg/ml) | > 600 | > 600 | **-** |

### Bsp. 7. : Einfluss verschiedener Chelatoren auf das Testsystem

Die Gerinnungszeit wurde mit einem Koagulometer KC4 (Amelung, Lemgo. Deutschland) bestimmt. 40 µl FV Mangelplasma, 10 µl Plasmaprobe und 50 µl 2 U/ml Protac®, 1U/ml RVV-V und 0.1 mg/ml Kephalin wurden 20 Minuten bei 37° C inkubiert. Die Gerinnung wurde durch die Zugabe von 50 µl 5 µg/ ml Prothrombinaktivator aus Notechis scutatus scutatus-Gift in 15 mM EDTA, Citrat oder Oxalat ausgelöst. Alle Reagenzien wurden in 50 mM Hepes, pH 7.5 gelöst. Die Gerinnungszeit wird in Anwesenheit und Abwesenheit von Protac® bestimmt. Aus der Gerinnungszeit mit Protac® und ohne Protac® wird ein Verhältnis (Ratio) gebildet (Tabelle 8).

Die Resultate zeigen, wie der Zusatz an verschiedenen Chelatoren die FV-Abhängigkeit verstärkt.

**Tabelle 8**

| | Gerinnungszeit + Protac® [s] | Gerinnungszeit - Protac® [s] | **Quotient +/- Protac®** |
|---|---|---|---|
| 1. 15 mM EDTA | 162.1 | 62.5 | **2.59** |
| 2. 15 mM Citrat | 115.4 | 55.8 | **2.07** |
| 3. 15 mM Oxalat | 92.8 | 49.1 | **1.89** |
| 4. Ohne Zusätze | 76.4 | 46.3 | **1.65** |

### Bsp. 8: Bestimmung der APC Resistenz mit chromogenem Substrat

Die Extinktionsänderung pro Minute wird bei 405 nm photometrisch bestimmt (Perkin Elmer UV/VIS LAMBDA BIO 10). 40 µl FV Mangelplasma, 10 µl Plasmaprobe und 50 µl 10 µg/ml APC, 10 U/ml RVV-V und 0.1 mg/ml Kephalin wurden 8 Minuten bei 37° C inkubiert. In einem 2. Schritt wurde nach den 8 min bei 37° C 50 µl des obigen aktivierten Gemisches zu 750 µl 50 mM Hepes, pH 7.5 , 100 µl 4 mM Tos-Gly-Pro-Arg-pNA-AcOH (Pefachrom TH) und 100 µl 5 µg/ ml Prothrombinaktivator aus Notechis scutatus scutatus-Gift zugegeben und die Extinktionsänderung bei 405 nm gemessen (Tabelle 9). Alle Reagenzien wurden in 50 mM Hepes, 15 mM EDTA bei pH 7.5 gelöst. Gemessen wird in Anwesenheit und Abwesenheit von APC. Aus der Extinktionsänderung/Minute ohne APC (- APC) und mit APC (+ APC) wird wiederum eine Ratio gebildet.

Das Umsetzen des chromogenen Substrates wird bei Normalplasma in Anwesenheit von APC stärker herabgesetzt, da hier normaler FV durch APC abgebaut wird und daher weniger Thrombin gebildet wird, das das chromogene Substrat spaltet. Die Extinktionsänderung/Minute bei Normalplasma in Anwesenheit von APC ist daher tiefer als beim Vorhandensein von APC-Resistenz.

Die erhaltenen Resultate zeigen, dass auch die chromogene Bestimmung der APC-Resistenz nach der Erfindung möglich ist und die Normalplasmen in jedem Fall von den Heterozygoten unterschieden werden können. Auch in diesem Beispiel wird wieder auf die Zugabe von Calciumionen verzichtet.

**Tabelle 9**

| | Normalplasma | | Heterozygoter FV Leiden | |
|---|---|---|---|---|
| | + APC | - APC | +APC | - APC |
| ΔE/min | 0.036 | 0.115 | 0.105 | 0.183 |
| Ratio (- APC/ + APC) | 3.18 | | 1.74 | |

### Bsp. 9: Heparineinfluss auf das Testsystem

Die Gerinnungszeit wurde mit einem Koagulometer KC4 micro (Amelung, Lemgo, Deutschland) bestimmt. 40 µl FV Mangelplasma (mit oder ohne Polybrene), 10 µl Plasmaprobe und 50 µl 2 U/ml Protac®, 1U/ml RVV-V und 0.1 mg/ml Kephalin wurden 20 Minuten bei 37° C inkubiert. Die Gerinnung wurde durch die Zugabe von 50 µl 5 µg/ ml Prothrombinaktivator aus Notechis scutatus scutatus-Gift in 15 mM EDTA ausgelöst. Alle Reagenzien wurden in 50 mM Hepes, pH 7.5 gelöst. Die Gerinnungszeit wird in Anwesenheit und Abwesenheit von Protac® bestimmt. Aus der Gerinnungszeit mit Protac® und ohne Protac® wurde eine Ratio gebildet (Tabelle 10).

Die Resultate zeigen, dass Heparin in therapeutischen Konzentrationen im vorgestellten Test keinen Einfluss auf die Ratio hat. Der Zusatz von Polybrene im FV-Mangelplasma hat auf die Ratio auch keinen Einfluss, doch werden dadurch die Gerinnungszeiten verlängert.

**Tabelle 10**

| Plasmaproben ohne Heparin | | | | | | |
|---|---|---|---|---|---|---|
| Plasmaprobe | FV Mangelplasma ohne Heparin-Antagonist | | | FV Mangelplasma mit Heparin-Antagonist (Chromogenix®) | | |
| | + Protac | - Protac | Ratio | + Protac | - Protac | Ratio |
| Normalplasma | 127.0 | 56.4 | 2.25 | 185.4 | 80.9 | 2.29 |
| Heterozygoter FV Defekt | | | | 70.6 | 65.9 | 1.07 |
| Heterozygoter FV Defekt | | | | 60.5 | 59.4 | 1.02 |
| Heterozygoter FV Defekt | | | | 81.2 | 69.8 | 1.16 |
| Heterozygoter FV Defekt | | | | 103.4 | 78.6 | 1.32 |
| Heterozygoter FV Defekt | | | | 124.9 | 94.5 | 1.32 |
| Heterozygoter FV Defekt | | | | 92.8 | 69.9 | 1.65 |

| Heparinisiertes Plasma: (0.5 oder 1.0 UI Heparin/ml Plasmaprobe). | | | | | | |
|---|---|---|---|---|---|---|
| Plasmaprobe | FV Mangelplasma ohne Heparin-Antagonist | | | FV Mangelplasma mit Heparin-Antagonist | | |
| | + Protac | - Protac | Ratio | + Protac | - Protac | Ratio |
| Heterozygoter FV Defekt | | | | | | |
| 0.5 UI Heparin | 70.3 | 55.8 | 1.26 | 84.9 | 66.8 | 1.27 |
| 1.0 UI Heparin | 70.9 | 56.6 | 1.25 | 102.2 | 73.0 | 1.40 |
| Heterozygoter FV Defekt | | | | | | |
| 0.5 UI Heparin | 88.1 | 62.2 | 1.42 | 113.7 | 83.9 | 1.36 |
| 1.0 UI Heparin | 86.8 | 62.2 | 1.40 | 119.3 | 83.0 | 1.44 |
| Normal | | | | | | |
| 0.5 UI Heparin | 170.4 | 61.6 | 2.77 | 206.3 | 84.2 | 2.45 |
| 1.0 UI Heparin | 179.0 | 62.4 | 2.87 | 206.3 | 92.1 | 2.24 |

## Patentansprüche

1. Verfahren zum qualitativen und/oder quantitativen Nachweis von Störungen im Protein C-System, **dadurch gekennzeichnet, dass** man eine Plasmaprobe mit einem Protein C-Aktivator und/oder APC inkubiert, die Gerinnung durch Zugabe eines calciumunabhängigen, jedoch FV-abhängigen, Prothrombinaktivators ohne Zusatz von Ca²⁺-lonen zum Testgemisch auslöst, die Gerinnungszeit misst, mit der Gerinnungszeit mit oder ohne Protein C-Aktivator und/oder APC einer Referenzplasmaprobe vergleicht und daraus die Störung im Protein C-System nachweist.

2. Verfahren nach Patentanspruch 1, **dadurch gekennzeichnet, dass** man als Referenzplasma ein Normalplasma verwendet.

3. Verfahren nach Patentanspruch 1, **dadurch gekennzeichnet, dass** man als Referenzplasma einen Teil der zu untersuchenden Plasmaprobe verwendet.

4. Verfahren nach einem der Patentansprüche 1-3, **dadurch gekennzeichnet, dass** man die Störung im Protein C-System durch Bildung eines Quotienten zwischen der Gerinnungszeit der Plasmaprobe und dem Referenzplasma nachweist.

5. Verfahren nach einem der Patentansprüche 1-4, **dadurch gekennzeichnet, dass** man die Messung mit synthetischen Substraten chromogen oder fluorogen oder amperogen durchfuhrt.

6. Verfahren nach einem der Patentansprüche 1-5, **dadurch gekennzeichnet, dass** man Plasmaproben verwendet, die mit Citrat, Oxalat oder EDTA vorbehandelt wurden.

7. Verfahren nach einem der Patentansprüche 1-6, **dadurch gekennzeichnet, dass** man als Protein C-Aktivator Gift oder mindestens eine Giftfraktion der Schlangen Agkistrodon contortrix und ihrer Unterarten, Agkistrodon piscivorus und ihrer Unterarten, Agkistrodon bilineatus und ihrer Unterarten und/oder Agkistrodon halys und ihrer Unterarten verwendet.

8. Verfahren nach einem der Patentansprüche 1-7, **dadurch gekennzeichnet, dass** man als Protein C-Aktivator Protac® verwendet.

9. Verfahren nach einem der Patentansprüche 1-8, **dadurch gekennzeichnet, dass** als Prothrombinaktivator Schlangengifte mit caiciumunabhängiger Prothrombinaktivatoraktivität in Form von Rohgift oder gereinigten Rohgrftfraktionen oder calciumunabhängige Prothrombinaktivatoren aus Mikroorganismen. mit natürlichen oder rekombinierten Genomen verwendet werden.

10. Verfahren nach Patentanspruch 9, **dadurch gekennzeichnet, dass** als Schlangengift mit calciumunabhängiger Prothrombinaktivatoraktivität dasjenige der Art der Elapidae, der Schlangen Notechis scutatus scutatus, Notechis, ater niger, Notechis ater humphreysi, Notechis ater serventyi, Notechis flinders, Notechis occidentalis, Tropidechis carinatus, Cryptophis nigrescens, Hoplocephalus stephensii und/oder Pseudechis porphyriacus verwendet wird.

11. Verfahren nach einem der Patentansprüche 1-10, **dadurch gekennzeichnet, dass** man Phospholipide, Protein C, Chelatoren oder Chelatoren-Analoga, Mangelplasma, Protein S, Faktor VIII, einen Faktor V-Aktivator und/oder Heparinantagonisten dem Testgemisch zufügt.

12. Verfahren nach Patentanspruch 11, **dadurch gekennzeichnet, dass** man als Mangelplasma Faktor V-, Protein C- oder Protein S-Mangelplasma dem Testgemisch zufügt.

13. Verfahren nach Patentanspruch 11 oder 12, **dadurch gekennzeichnet, dass** man Mangelplasma im Verhältnis zur Plasmaprobe in Mengen von 99% zu 1% bis 50% zu 50% verwendet.

14. Verfahren nach Patentanspruch 11, **dadurch gekennzeichnet, dass** als Faktor V-Aktivator RVV-V aus Vipera russelli verwendet wird:

15. Verfahren nach Patentanspruch 11, **dadurch gekennzeichnet, dass** als Heparinantagonist Hexadimethrinbromid, Protaminsulfat oder heparinspaltende Enzyme verwendet werden.

16. Verfahren nach Patentanspruch 11, **dadurch gekennzeichnet, dass** als Chetator Citrat, Oxalat, EGTA und/oder EDTA verwendet werden.

17. Anwendung des Verfahrens nach einem der Patentansprüche 1-16, zum qualitativen Nachweis und/oder der quantitativen Bestimmung von aktiviertem Blutgerinnungsfaktor V mit erhöhter Stabilität gegenüber dem Abbau durch aktiviertes Protein C in einer Plasmaprobe im Falle von FV Leiden.

18. Testkit zur Durchführung des Verfahrens nach einem der Patentansprüche 1-16 enthaltend APC oder einen Protein C-Aktivator, einen calciumunabhängigen, jedoch FV-abhängigen Prothrombinaktivator und gegebenenfalls einen Faktor V-Aktivator.

19. Testkit nach Anspruch 18, enthaltend Protac® als Protein C-Aktivator.

20. Testkit nach Anspruch 18 oder 19, enthaltend Phospholipide, Faktor V-Mangelplasma, einen Ca-Komplexbildner, und gegebenenfalls, ein oder mehrere Referenzplasmen.

21. Testkit nach Anspruch 20, enthaltend EDTA als Ca-Komplexbildner.

22. Verwendung des Testkit nach einem der Ansprüche 18-21 zum qualitativen Nachweis und/oder der quantitativen Bestimmung von aktiviertem Blutgerinnungsfaktor V mit erhöhter Stabilität gegenüber dem Abbau durch aktiviertes Protein C in einer Plasmaprobe, im Falle von Faktor V Leiden, oder von Störungen im Protein C- oder Protein S-System.

## Claims

1. A method for the qualitative and/or quantitative determination of disorders in the protein C system, comprising incubating a plasma sample with a protein C activator and/or APC, triggering off the coagulation by the addition of a calcium-independent, but FV-dependent, prothrombin activator without addition of calcium ions to the test mixture, measuring the clotting time, comparing the latter with the clotting time with or without protein C activator and/or APC of a reference plasma sample and detecting the disturbance in the protein C system therefrom.

2. A method according to claim 1, wherein the reference plasma is a normal plasma.

3. A method according to claim 1, wherein the reference plasma is a part of the plasma sample to be investigated.

4. A method according to one of the claims 1-3, wherein the disorder in the protein C system is detected by forming a quotient with the clotting time of the plasma sample and the reference plasma.

5. A method according to one of the claims 1-4, wherein the measurement is carried out with synthetic chromogenic, fluorogenic or amperogenic substrates.

6. A method according to one of the claims 1-5, wherein the used plasma samples were pre-treated with citrate, oxalate or EDTA.

7. A method according to one of the claims 1-6, wherein the protein C activator is a venom or at least a venom fraction from the snakes Agkistrodon contortrix and its subspecies, Agkistrodon piscivorus and its subspecies, Agkistrodon bilineatus and its subspecies and/or Agkistrodon halys and its subspecies.

8. A method according to one of the claims 1-7, wherein the used protein C activator is Protac®.

9. A method according to one of the claims 1-8, wherein the prothrombin activator is a snake venom with calcium-independent prothrombin activator activity in the form of crude venom or a purified crude venom fraction or a calcium-independent prothrombin activator from microorganisms with natural or recombinant genomes.

10. A method according to claim 9, wherein the snake venom with calcium-independent prothrombin activator activity is a venom from an Elapidae species, from the snakes Notechis scutatus scutatus, Notechis ater niger, Notechis ater humphreysi, Notechis ater serventyi, Notechis flinders, Notechis occidentalis, Tropidechis carinatus, Cryptophis nigrescens, Hoplocephalus stephensii and/or Pseudechis prophyriacus.

11. A method according to one of the claims 1-10, comprising adding to the test mixture phospholipids, protein C, chelating agents or chelating agent analogues, deficient plasma, protein S, factor VIII, a factor V activator and/or heparin antagonists.

12. A method according to claim 11, comprising adding to the test mixture factor V-, protein C- or protein S-deficient plasma as the deficient plasma.

13. A method according to claim 11 or 12, wherein the deficient plasma is used in quantities of 99% to 1% up to 50% to 50% in relation to the plasma sample.

14. A method according to claim 11, wherein the factor V activator is RW-V from Vipera russelli.

15. A method according to claim 11, wherein the heparin antagonist is hexadimethrine bromide, protamine sulfate or a heparin-splitting enzyme.

16. A method according to claim 11, wherein the chelating agent is citrate, oxalate, EGTA and/or EDTA.

17. Use of the method according to one of the claims 1-16, for the qualitative detection and/or quantitative determination of activated blood clotting factor V with increased stability to decomposition by activated protein C in a plasma sample in case of FV Leiden.

18. A test kit for applying the method according to one of the claims 1-16, comprising APC or a protein C activator, a calcium-independent, but FV-dependent, prothrombin activator and optionally a factor V activator.

19. A test kit according to claim 18, comprising Protac® as the protein C activator.

20. A test kit according to claim 18 or 19, comprising phospholipids, factor V-deficient plasma, a Ca-complexing agent and, optionally, one or more reference plasmas.

21. A test kit according to claim 20, comprising EDTA as the Ca-complexing agent.

22. Use of the test kit according to one of the claims 18-21 for the qualitative detection and/or quantitative determination of activated blood-clotting factor V with increased stability to decomposition by activated protein C in a plasma sample, in case of factor V Leiden, or of disorders in the protein C or protein S system.

## Revendications

1. Méthode pour la détection qualitative et/ou quantitative de troubles du système de la protéine C, **caractérisée en ce que** l'on incube un échantillon de plasma avec un activateur de protéine C et/ou de la PCA, que l'on déclenche la coagulation par l'addition d'un activateur de prothrombine indépendant du calcium mais dépendant du FV sans ajouter d'ions calcium au mélange de test, que l'on mesure le temps de coagulation, que l'on compare ce dernier avec le temps de coagulation avec ou sans activateur de protéine C et/ou de PCA d'un échantillon de plasma de référence et que l'on détecte la perturbation du système de la protéine C.

2. Méthode selon la revendication 1, **caractérisée en ce que** le plasma de référence est un plasma normal.

3. Méthode selon la revendication 1, **caractérisée en ce que** le plasma de référence est une partie de l'échantillon de plasma à étudier.

4. Méthode selon l'une des revendications 1-3, **caractérisée en ce que** le trouble du système de la protéine C est détecté par la formation d'un quotient entre le temps de coagulation de l'échantillon de plasma et le plasma de référence.

5. Méthode selon l'une des revendications 1-4, **caractérisée en ce que** la mesure est réalisée à l'aide de substrats synthétiques chromogènes, fluorogènes ou ampérogènes.

6. Méthode selon l'une des revendications 1-5, **caractérisée en ce que** l'on utilise des échantillons de plasma prétraités avec du citrate, de l'oxalate ou de l'EDTA.

7. Méthode selon l'une des revendications 1-6, **caractérisée en ce que** l'on utilise comme activateur de protéine C du venin ou au moins une fraction du venin des serpents Agkistrodon contortrix et ses sous-espèces, Agkistrodon piscivorus et ses sous-espèces, Agkistrodon bilineatus et ses sous-espèces et/ou Agkistrodon halys et ses sous-espèces.

8. Méthode selon l'une des revendications 1-7, **caractérisée en ce que** l'on utilise Protac® comme activateur de protéine C.

9. Méthode selon l'une des revendications 1-8, **caractérisée en ce que** l'on utilise comme activateur de prothrombine soit un venin de serpent ayant une activité d'activateur de prothrombine indépendant du calcium sous la forme de venin brut ou de fractions de venin brut purifiées, soit un activateur de prothrombine indépendant du calcium provenant de micro-organismes avec des génomes naturels ou recombinants.

10. Méthode selon la revendication 9, **caractérisée en ce que** le venin de serpent ayant une activité d'activateur de prothrombine indépendant du calcium est un venin de l'espèce Elapidae, des serpents Notechis scutatus scutatus, Notechis ater niger, Notechis ater humphreysi, Notechis ater serventyi, Notechis flinders, Notechis occidentatis, Tropidechis carinatus, Cryptophis nigrescens, Hoplocephalus stephensii et/ou Pseudechis prophyriacus.

11. Méthode selon l'une des revendications 1-10, **caractérisée en ce que** l'on ajoute au mélange de test des phospholipides, de la protéine C, des chélatants ou des analogues de chélatants, du plasma déficient, de la protéine S, du facteur VIII, un activateur de facteur V et/ou des antagonistes d'héparine.

12. Méthode selon la revendication 11, **caractérisée en ce que** le plasma déficient ajouté au mélange de test est du plasma déficient en facteur V, en protéine C ou en protéine S.

13. Méthode selon la revendication 11 ou 12, **caractérisée en ce que** l'on utilise du plasma déficient dans des proportions de 99% à 1% jusqu'à 50%-50% par rapport à l'échantillon de plasma.

14. Méthode selon la revendication 11, **caractérisée en ce que** l'activateur de facteur V est du RVV-V de Vipera russelli.

15. Méthode selon la revendication 11, **caractérisée en ce que** l'antagoniste d'héparine est du bromure d'hexadiméthrine, du sulfate de protamine ou une enzyme scindant l'héparine.

16. Méthode selon la revendication 11, **caractérisée en ce que** le chélatant est du citrate, de l'oxalate, de l'EGTA et/ou de l'EDTA.

17. Application de la méthode selon l'une des revendications 1-16, pour la détection qualitative et/ou le dosage quantitatif du facteur de la coagulation sanguine V activé ayant une stabilité accrue par rapport à la décomposition par la protéine C activée dans un échantillon de plasma en cas de FV Leiden.

18. Trousse de test pour l'application de la méthode selon l'une des revendications 1-16, contenant de la PCA ou un activateur de protéine C, un activateur de prothrombine indépendant du calcium mais dépendant du FV et éventuellement un activateur de facteur V.

19. Trousse de test selon la revendication 18, contenant Protac® comme activateur de protéine C.

20. Trousse de test selon la revendication 18 ou 19, contenant des phospholipides, du plasma déficient en facteur V, un agent complexant le calcium et éventuellement un ou plusieurs plasmas de référence.

21. Trousse de test selon la revendication 20, contenant de l'EDTA comme agent complexant le calcium.

22. Utilisation de la trousse de test selon l'une des revendications 18-21 pour la détection qualitative et/ou le dosage quantitatif du facteur de coagulation V activé ayant une stabilité accrue par rapport à la décomposition par la protéine C activée dans un échantillon de plasma en cas de facteur V Leiden ou de troubles du système de la protéine C ou de la protéine S.
